(19) 

Europäisches
Patentamt
European
Patent Office
Office européen
des brevets

(11) **EP 3 096 253 A1**

(12) **EUROPEAN PATENT APPLICATION**

(43) Date of publication:
**23.11.2016 Bulletin 2016/47**

(51) Int Cl.:
***G06F 19/00*** *(2011.01)*

(21) Application number: **15168068.3**

(22) Date of filing: **19.05.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Universidad de Vigo**
**36310 Vigo (Pontevedra) (ES)**

(72) Inventors:
• **Hermida Domínguez, Ramón Carmelo**
**15886 Oza-Teo (A Coruña) (ES)**

• **Fernández Bernárdez, José Ramón**
**36317 Vigo (Pontevedra) (ES)**
• **Mojón Ojea, Artemio**
**36206 Vigo (Pontevedra) (ES)**
• **Ayala García, Diana Elva**
**15886 Oza-Teo ( A Coruña) (ES)**

(74) Representative: **Carvajal y Urquijo, Isabel et al**
**Clarke, Modet & Co.**
**Suero de Quiñones, 34-36**
**28002 Madrid (ES)**

(54) **SYSTEM, COMPUTER-IMPLEMENTED METHOD AND COMPUTER PROGRAM PRODUCT FOR INDIVIDUALIZED MULTIPLE-DISEASE QUANTITATIVE RISK ASSESSMENT**

(57) System for individualized multiple-disease quantitative risk assessment, comprising:
- a database (110) storing clinical data of patients with prognostic variables including biological signals (118);
- an analysis module (114) for processing the biological signals (118) for a given patient to automatically calculate prognostic parameters (120) of diagnostic relevance for said patient;
- a risk-evaluation module (116) for estimating, using a risk-evaluation model that considers predictor variables among the prognostic variables stored in the database (110) and the prognostic parameters (120) calculated by the analysis module (114), an individualized hazard ratio (122) of having an event or developing a medical condition within a given period of time for the given patient; and
- an individualized multiple-disease quantitative risk assessment module (124) for computing, based on the risk-evaluation model and specified thresholds for each of the predictor variables, a quantification of the risk (126) for the given patient for each of the events or medical conditions under evaluation.

EP 3 096 253 A1

**Description**

*Technical field*

Disclosed embodiments relate to web-enabled systems for clinical research. Specifically, it is related to web-enabled systems for individualized multiple risk assessment.

*Background*

The static threshold values for conventional single-time laboratory data and other physiological variables of clinical interest, including blood pressure (BP) and heart rate, currently used for diagnosis, risk stratification, and clinical decision-making regarding treatment do not take into account the predictable 24h pattern in those variables. For example, the diagnosis of hypertension typically is based on a limited number of daytime clinic BP measurements obtained in the physician's office, occasionally supplemented by wake-time patient self-assessments at home and work and thus totally omitting the value of BP during sleep at night. However, correlation between BP level and target organ damage and cardiovascular disease (CVD) risk has been consistently shown to be higher for ambulatory BP monitoring (ABPM) than clinic BP measurements. An important advantage of around-the-clock ABPM is thorough description and quantification of the mostly predictable daily BP variation that results from the interrelationship of various internal and external time-of-day influences: (i) rest-activity associated changes in behavior (including activity routine and level, meal timings and content, mental stress, and posture); (ii) day-night divergence in ambient temperature, humidity, and noise; and (iii) circadian (~24h) variation in neuroendocrine, endothelial, BP-modulating peptide, and hemodynamic parameters, e.g., plasma noradrenaline and adrenaline (autonomic nervous system), atrial natriuretic and calcitonin gene-related peptides, and renin, angiotensin, and aldosterone (renin-angiotensin-aldosterone system, RAAS).

In many, but not all, persons with normotension or uncomplicated essential hypertension, systolic (SBP) and diastolic BP (DBP) fall to lowest levels during nighttime sleep, rise with morning awakening and elevate to highest values during daytime activity. Specific features of this daily BP pattern have been explored as biomarkers or mediators of target tissue injury and triggers of and risk factors for CVD events -- angina pectoris, myocardial infarction, cardiac arrest, sudden cardiac death, pulmonary embolism -- and cerebrovascular events -- ischemic and hemorrhagic stroke. The fall of BP during sleep is commonly quantified by the sleep-time relative BP decline (percent decrease in mean BP during night-time sleep relative to the mean BP during daytime activity). Numerous ABPM studies consistently substantiate the incidence of end-organ injury and fatal and non-fatal CVD events are significantly associated with blunted sleep-time relative BP decline (non-dipping), not only in hypertensive patients, but also in normotensive individuals. Furthermore, various independent prospective studies demonstrate CVD events are better predicted by the asleep than awake or 24h BP means.

Overall, these prospective studies demonstrate sleep-time hypertension and non-dipping BP patterning constitute significant CVD risk factors that are independent of the daytime and 24h BP means. Nonetheless, the findings of these previous studies may be imprecise because of inherent limitations of their investigative methods. All previous studies addressing the merit of ABPM for predicting CVD risk, except the Ambulatory Blood Pressure Monitoring for Prediction of Cardiovascular Events (MAPEC study) discussed below, relied upon only a single, low-reproducible, 24h ABPM evaluation per participant at study inclusion. Such study design is unsound because it presumes features of the baseline-determined ambulatory BP pattern are maintained without alteration during the many years of follow-up in spite of BP-lowering therapy, aging, and/or development of target organ damage and concomitant morbidity. Additional limitations of most previous ABPM studies are: (i) frequent use of arbitrarily fixed clock hours to define morning awakening and evening bedtime, resulting in daytime and nighttime BP means that do not accurately represent awake and asleep BP, because they are calculated without assessing and taking into account the actual rest and activity spans of each participant; and (ii) analysis of the prognostic value of dipping status and nighttime BP mean without proper adjustment for the daytime BP mean. Moreover, lack of periodic multiple ABPM evaluations in all previous reported studies precluded the opportunity to evaluate the potential reduction in CVD risk associated with modification of prognostic parameters, i.e., either increase of sleep-time relative BP decline towards a more normal dipper patterning or, more specifically, reduction of asleep BP mean. Incorporation of periodic (at least annual) ABPM evaluations during follow-up -- as in the MAPEC study -- clearly establishes: (i) features of the daily BP pattern undergo change over time; and (ii) therapeutic reduction of the asleep BP mean and increase of the sleep-time relative BP decline towards normal dipping profile lessen CVD risk. These findings are additionally substantiated for MAPEC study cohorts with type 2 diabetes, chronic kidney disease (CKD), and resistant hypertension. Interestingly, sleep-time hypertension and/or the non-dipper/riser BP pattern are highly prevalent in individuals with secondary and resistant hypertension, as well as those diagnosed with metabolic syndrome, diabetes, CKD, obstructive sleep apnea and other sleep disorders, among other medical conditions, leading to the question as to if sleep-time BP alteration is just a consequence or if precedes these complications, opening the possibility to early risk assessment.

The same authors have previously proposed (US Pat No 8,428,965 B2) a system for clinical managing of CVD risk using ABPM and actigraphy. However, the objective of such system is to establish an individualized refined diagnosis of hypertension based on ABPM as a single influential CVD risk factor, and it is not intended to allow determination of an individualized hazard ratio (HR) for future occurrence of a fatal or non-fatal CVD events. On the other hand, other available CVD risk stratification systems (Framingham, SCORE, etc.) are limited to provide a classification of subjects in a stepwise, more qualitative than properly quantitative, risk scale using only traditional population-based risk factors (clinic BP, smoking, cholesterol, etc.) and totally disregarding the valuable prognostic information derived from ABPM. Furthermore, these CVD risk scales have additional major limitations: (i) they are limited to evaluate risk of some specific CVD events; for example, the Framingham scale was designed to predict only the risk of coronary heart disease, failing to provide information on the risk of other major CVD events, including stroke and heart failure; (ii) being based on specific populations, they can overestimate or underestimate risk in other populations; and (iii) they have never been developed to assess the risk of other conditions, such as diabetes and CKD, associated with a high prevalence of BP alteration.

*Disclosed embodiment*

Disclosed embodiments relate to a system and method for individualized multiple-disease risk assessment, including cardiovascular and cerebrovascular events, progression towards diabetes, renal disease and other pathologies.

In most clinical applications the main objective should be to evaluate, for any given person, his/her individualized hazard of having an event (e.g., myocardial infarction, stroke, angina pectoris, cardiac arrest) or developing a medical condition (e.g., hypertension, diabetes, CKD) within a given period of time. In such cases, for each specific endpoint of interest, e.g., total CVD events, coronary events, new-onset diabetes, etc., a Cox proportional-hazard model, with adjustment for significant confounding variables, can be readily used to estimate hazard ratios (with about 95% confidence intervals) for events associated with each tested potential prognostic parameter, either specific time-qualified measurements of physiological variables of clinical interest for their prognostic value and/or characteristics of their 24h predictable pattern. Once the hazard model is established, the quantitative individualized risk for any given subject can be established as a relative hazard ratio from a reference individual, that with physiological prognostic characteristics associated with lowest risk. The novel proposed ability to quantify an individualized hazard ratio for multiple risk assessment would enable not only estimating the hazard that such individual might indeed suffer CVD, metabolic, renal, and other events, but also assess, in subsequent evaluations during follow-up, the effects of therapeutic intervention on his/her individualized multiple hazard ratios.

In accordance with one aspect of the present invention there is provided a system for individualized multiple-disease quantitative risk assessment. The system comprises:

- a database storing clinical data of at least one patient, said clinical data comprising prognostic variables including at least a plurality of biological signals measured on the at least one patient;
- an analysis module for processing the biological signals stored in the database for a given patient through at least one statistical method and analysis technique, to automatically calculate prognostic parameters of diagnostic relevance for said patient;
- a risk-evaluation module for estimating, using a risk-evaluation model that considers predictor variables among the prognostic variables stored in the database and the prognostic parameters calculated by the analysis module, an individualized hazard ratio of having an event or developing a medical condition within a given period of time for the given patient; and
- an individualized multiple-disease quantitative risk assessment module for computing, based on the risk-evaluation model performed in the risk evaluation module and specified thresholds for each of the predictor variables incorporated in the risk-evaluation model, a quantification of the risk for the given patient for each of the events or medical conditions under evaluation.

The statistical method and analysis technique performed in the analysis module may include:

- Methods for computation of time-specified tolerance intervals and bands, wherein for a given lower and upper limit of a time-varying tolerance interval of the biological signal under analysis, diagnosis of disease for a given patient is established in the analysis module by calculating the total area of the patient's biological signal measurements above or below the lower and upper limits during the entire 24 hour period.
- Methods for time-series periodic evaluation that provide sensitivity diagnostic endpoints.
- Methods for time-series parameter estimation.

In a preferred embodiment the risk-evaluation module uses a Cox proportional-hazard model to estimate the hazard ratio for a given patient. The individualized hazard ratio is preferably computed as the ratio between the risk-evaluation models evaluated for the given patient and a reference individual with normative values for all the predictor variables.

The clinical data stored in the database may include laboratory data derived from blood, urine or saliva, current treatment of each patient, personal medical history, anthropometric information and personal habits.

The events or medical conditions under evaluation may include cardiovascular events, cerebrovascular events, progression towards diabetes, renal disease and/or sleep disorders.

In a preferred embodiment the system further comprises a server running a web-enabled graphical user interface to enable a user to authenticate and upload clinical data of different patients.

In accordance with a further aspect of the present invention there is provided a computer-implemented method for individualized multiple-disease quantitative risk assessment. The computer-implemented method comprises:

- retrieving, from a database storing clinical data of at least one patient comprising prognostic variables which include at least a plurality of biological signals measured on the at least one patient, biological signals for a given patient;

- processing the retrieved biological signals through at least one statistical method and analysis technique, to automatically calculate prognostic parameters of diagnostic relevance for said patient;

- estimating, using a risk-evaluation model that considers predictor variables among the prognostic variables stored in the database and the prognostic parameters calculated by the analysis module, an individualized hazard ratio of having an event or developing a medical condition within a given period of time for the given patient; and

- computing, based on the risk-evaluation model performed in the risk evaluation module and specified thresholds for each of the predictor variables incorporated in the risk-evaluation model, a quantification of the risk for the given patient for each of the events or medical conditions under evaluation.

The computer-implemented method may further comprise dynamically reevaluating the risk assessment for a given patient as new clinical data for said patient is stored in the database. This allows a follow-up of a given individual, for instance to evaluate the treatment-induced changes in individualized risk of multiple conditions.

In accordance with a yet further aspect of the present invention there is provided a computer program product for individualized multiple-disease quantitative risk assessment, wherein the computer program product comprises at least one computer-readable storage medium comprising a set of instructions stored therein which, when executed by a processor, causes the processor to:

- retrieve, from a database storing clinical data of at least one patient comprising prognostic variables which include at least a plurality of biological signals measured on the at least one patient, biological signals for a given patient;

- process the retrieved biological signals through at least one statistical method and analysis technique, to automatically calculate prognostic parameters of diagnostic relevance for said patient;

- estimate, using a risk-evaluation model that considers predictor variables among the prognostic variables stored in the database and the prognostic parameters calculated by the analysis module, an individualized hazard ratio of having an event or developing a medical condition within a given period of time for the given patient; and

- compute, based on the risk-evaluation model performed in the risk evaluation module and specified thresholds for each of the predictor variables incorporated in the risk-evaluation model, a quantification of the risk for the given patient for each of the events or medical conditions under evaluation.

*Brief Description of the Drawings*

A series of drawings which aid in better understanding the disclosed embodiments and which are expressly related with an embodiment, presented as a non-limiting example thereof, are very briefly described below.

Figure 1 illustrates a block diagram according to one embodiment.

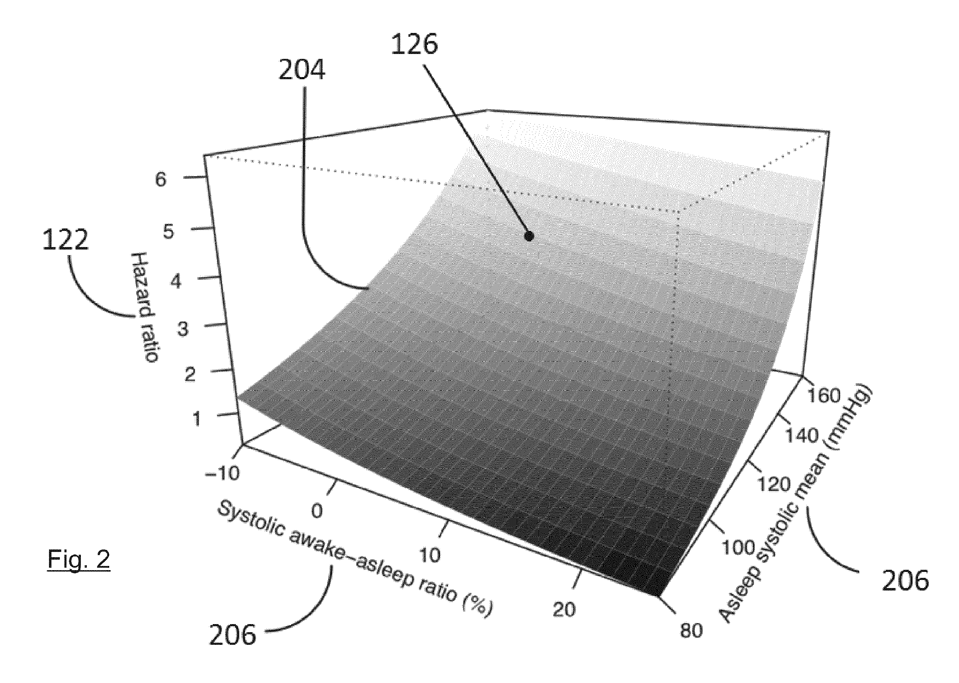

Figure 2 shows a three-dimensional risk function.

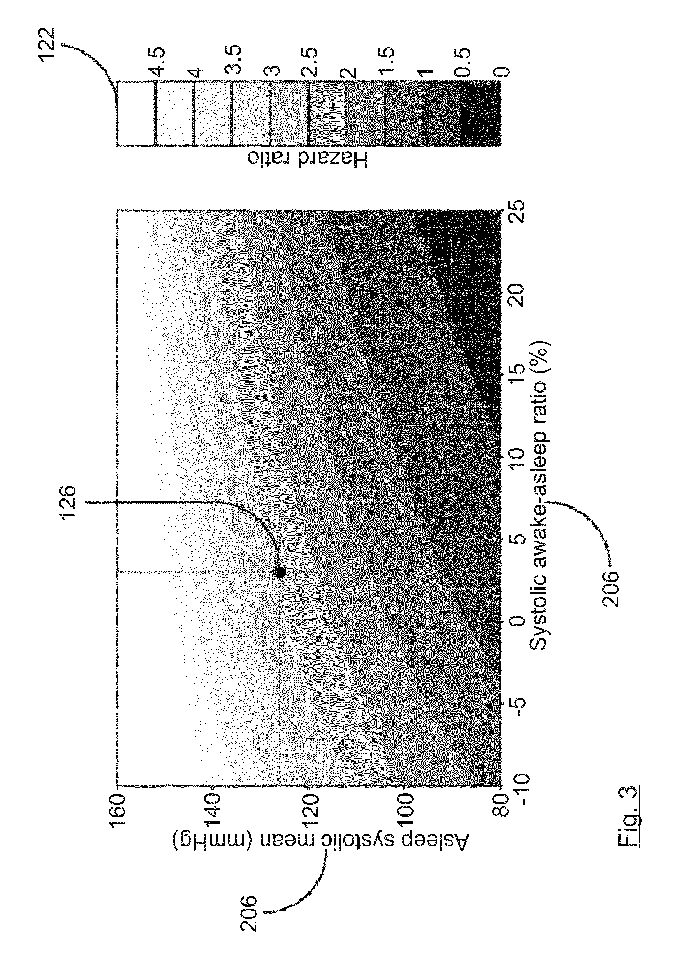

Figure 3 shows a two-dimensional risk function.

Figure 4 shows the adjusted HR for new-onset diabetes as a function of normal asleep BP whether the awake BP mean is normal or elevated.

Figure 5 shows the adjusted HR for new-onset diabetes as a function of normal asleep BP whether the awake BP mean is normal or elevated and the clinic BP is normal or elevated.

Figure 6 shows the adjusted HR for new-onset diabetes as a function of the dipper classification.

*Description of a Preferred Embodiment of the Invention*

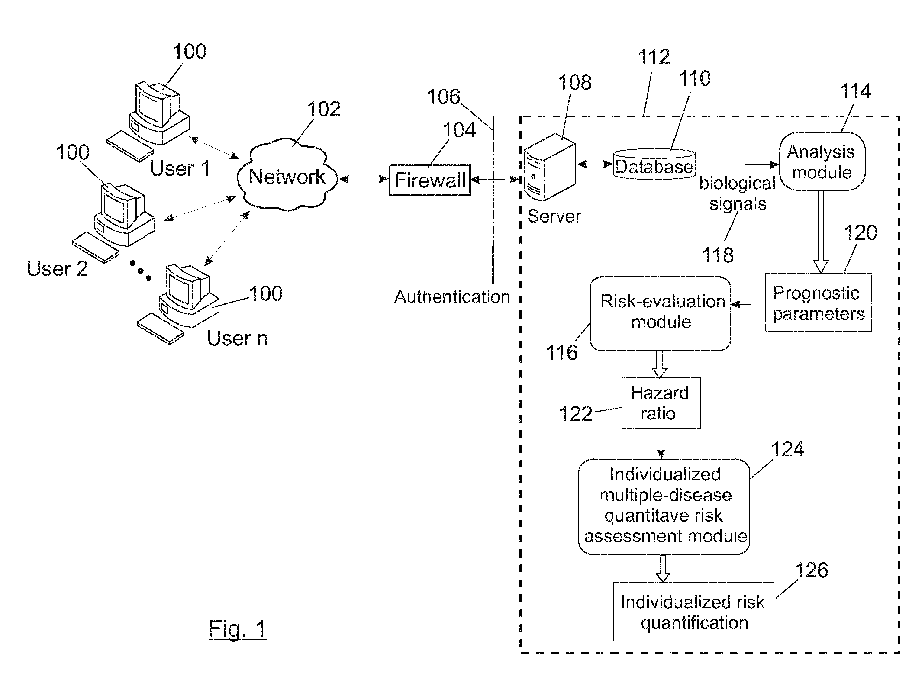

Figure 1 illustrates a block diagram according to one embodiment, in which a plurality of users **100** such as medical doctors, researchers, nurses, research coordinators, statisticians, and system administrators are connected to the system **112**, comprising at least a server **108** and a database **110**, through a computer network **102** such as the Internet or an intranet. Each user has a specific set of permissions associated with their profile that gives them access to specific datasets, analysis methods, and study results.

The server **108** receives and stores the data in a database **110** which is protected behind a firewall **104**.

Additionally, in order to grant access only to validated users and to maintain private the data from each user, the system includes a mechanism for secured authentication **106**. Although this embodiment refers to a web system including a web server **108**, it is not a requirement to employ a web interface to upload clinical data of patients into the database **110**. The clinical data can be, for instance, locally stored in the database, or they can be uploaded using different technical means.

Figure 1 shows the elements of the system **112** for individualized multiple risk assessment. Apart from the already-mentioned server **108** and database **110**, the system comprises:

- an analysis module **114** which calculates prognostic parameters **120**;
- a risk-evaluation module **116** which, using the prognostic variables stored in the database **110** and the prognostic parameters **120** calculated by the analysis module **114**, estimates a hazard ratio **122** of having an event or developing a medical condition within a given period of time; and
- an individualized multiple-disease quantitative risk assessment module **124**, which obtains a quantification of the risk **126** for a given individual for each of the events or medical conditions under evaluation.

The server **108** runs a web-enabled graphical user interface to enable a user to securely authenticate, securely upload clinical data and other required information, and securely navigate through a plurality of software modules for proper biomedical signal processing, mathematical modeling of predictable patterning, and individualized multiple risk assessment.

The database **110** stores in a memory storage device: a plurality of user profiles; specific trial protocols; different treatments or therapeutic recommendations according to the characteristics, risk or established medical conditions of the individualized patient; and clinical data of a plurality of individuals including, but not limited to, the following:

a) Personal medical history; anthropometric information (e.g. age, sex, height, weight, body mass index (BMI), waist perimeter, neck perimeter); personal habits (e.g. cigarette smoking, daily average of cigarettes, duration of smoking habit, alcohol consumption in units/week, and physical activity); mental state (including test scores and testing data).

b) Factors influencing prognosis, including for instance: risk factors (family history of premature vascular disease; hypertension, obstructive sleep apnea; atrial fibrillation; age of menopause in women); diabetes mellitus (including family history of diabetes in current and two previous generations); subclinical organ damage (including left ventricular hypertrophy, carotid wall thickening, carotid-femoral pulse wave velocity; ankle/brachial blood pressure index); congestive heart failure by NYHA classification; established cardiovascular, cerebrovascular, or renal disease; and other relevant diseases (including cancer).

c) Current treatment, which may include, for instance: hypertension medications; diabetes medications (oral antidiabetics or insulin); hypolipidemic medications; hipnotics and melatolin; stimulants; medications for movement disorders; seizure medications; antidepressants, antipsicotics, and mood medications; antiaggregation medications; oral contraceptives, etc. In all cases, duration of treatment, daily dose, and time-of-day of treatment is also registered for all reported medications. Additionally, treatment adherence and secondary effects (of any) of treatment are also registered at the database.

d) Laboratory data derived from blood (arterial or peripheral), urine or saliva, which may include for instance data on the following variables: glucose, HbA1c, total cholesterol, HDL-cholesterol, LDL-cholesterol, triglycerides, serum creatinine, uric acid, total proteins, albumin, globulin, GOT, GPT, GGT, sodium, potassium, calcium, fibrinogen, 25-OH vitamin D, leukocytes, erythrocytes, hemoglobin, hematocrit, platelets, lymphocytes, monocytes, basophiles, erythrocyte sedimentation velocity, reactive C protein, renin, angiotensin, aldosterone, cortisol, TSH, T3, T4, homocisteine, catecholamines, melatonin, glomerular filtration rate, urinary albumin excretion, albumin/creatinine ratio, mRNA.

e) A variety of biological signals measured along time in either room-restricted or fully ambulatory conditions with appropriate sensors or devices. The biological signals may include, for instance, any of the following: BP, actigraphy, body position and activity (BA), oxygen saturation (SaO2), heart rate, respiratory rate (RR), body temperature (BT), and long-term electrocardiogram (ECG), electroencephalogram (EEG), electrooculogram (EOG), and electromyogram (EMG).

The database **110** allows multiple evaluation of the same individual, i.e., all studied variables might be measured on different occasions during follow-up of a given individual, allowing prospective clinical monitoring of patients, to assess clinically relevant changes in all evaluated diagnostic and prognostic parameters over time, and to evaluate the treatment-induced changes in individualized risk of multiple conditions.

The database **110** allows registering the occurrence of new clinical conditions and events during follow-up (including cardiovascular events, cerebrovascular events, new-onset diabetes, new-onset and progression of renal disease, new-onset sleep disorders, etc.). This newly registered information will, in turn, allow systematic reevaluation and improvement of the mathematical procedures for risk assessment.

The dynamic structure of the database **110** allows development of customized solutions for potential specific clients (clinical sites, insurance companies, government agencies, etc.). This customized solution allows keeping the medical history recording with specific requirements set up by the client as well as generating standardized clinical reports for assessment and quantification of either one or multiple risks for medical conditions and events.

The system **112** also comprises an analysis module **114** which performs a plurality of statistical methods and analysis techniques for processing the biological signals **118** stored in the database **110**, specifically designed to automatically calculate specific prognostic parameters **120** or indices of diagnostic/prognostic relevance. The analysis module may be incorporated into the server **108**, implemented for instance as a server processor. It may also be implemented in a separate entity with processing means.

The static threshold values for conventional single-time determined physiological variables of clinical interest, including BP, BA, RR, BT, and $SaO_2$, currently used for diagnosis and clinical decision-making regarding treatment do not take into account the predictable circadian (and other frequencies) pattern in those variables. For example, the diagnosis of hypertension typically is based on a limited number of daytime clinic BP measurements obtained in the physician's office.

The development of automatic instrumentation for non-invasive ABPM, however, made it possible thorough description and quantification of the mostly predictable daily BP variation that results from the interrelationship of various internal and external time-of-day influences: (i) rest-activity associated changes in behavior (including activity routine and level, meal timings and content, mental stress, and posture); (ii) day-night divergence in ambient temperature, humidity, and noise; and (iii) circadian (~24h) variation in neuroendocrine, endothelial, BP-modulating peptide, and hemodynamic parameters, e.g., plasma noradrenaline and adrenaline (autonomic nervous system), atrial natriuretic and calcitonin gene-related peptides, and renin, angiotensin, and aldosterone (renin-angiotensin-aldosterone system, RAAS). Extensive research has demonstrated predictable circadian variability in plasma renin activity, angiotensin-converting enzyme, angiotensin I and II, aldosterone, atrial natriuretic peptide, and catecholamines.

Circadian rhythms in autonomic nervous system function are well known; sympathetic tone is dominant during the diurnal activity span, while vagal tone is dominant during most of the nighttime sleep span. This day-night oscillation of the autonomic nervous system is strongly linked to the sleep-wake circadian rhythm and plays a dominant role in the observed 24h BP variation in both normotension and uncomplicated essential hypertension. In diurnally active persons, the plasma level of norepinephrine and epinephrine is highest in the morning, during the initial hours of daytime activity and lowest during nocturnal sleep. Urinary catecholamine excretion also exhibits marked circadian rhythmicity of comparable phasing. The relationship between the 24h variation in plasma dopamine and norepinephrine/epinephrine levels is strong, indicating a dopaminergic modulation of the circadian rhythm of sympathetic nervous system activity. Most interesting, angiotensin and melatonin, both characterized by highly predictable 24h patterning, present opposite roles in cardiovascular and metabolic pathophysiology, as extensively documented. The mechanisms by which melatonin antagonizes angiotensin II in cardiovascular and metabolic diseases comprise its antihypertensive, antioxidant, and anti-inflammatory actions.

The specific analysis techniques which are performed in the analysis module **114** include:

1. Methods for computation of time-specified tolerance intervals and bands. The static diagnostic thresholds currently used in most clinical diagnostic applications might be replaced by a time-qualified reference limit reflecting the mostly predictable physiologic variability during the 24h. Once the time-varying threshold, given by a lower and upper limit of a tolerance interval, is available, diagnosis of disease might be established by calculating the total area of any given subject's analyzed variable (e.g., BP, angiotensin, glucose) of the patient's measurements (among the biological signals **118**) above the threshold during the entire 24h period. The area of excess, as well as the duration of excess (% time of excess, defined as the percentage time during the 24h when the analyzed variable of the test subject exceeds the upper limit of the tolerance interval), can then be used as nonparametric endpoints for prevention or diagnosis of overt disease.

2. Methods for periodic time-series evaluation that provide sensitivity diagnostic/prognostic endpoints. Methods of periodic regression are fully applicable to non-sinusoidal shaped time series data consisting of values distributed at equal or unequal intervals. The method produces estimates of the rhythm-adjusted time series mean or MESOR (Midline Estimating Statistic Of Rhythm, i.e., average value of the rhythmic function fitted to the data), as well as the amplitude (one-half the extent of the temporal variability explainable by rhythmicity), and acrophase (crest time expressed as a lag in time from a designated reference) for every significant fitted component of given periods. When the waveform shape of the rhythm is best approximated by a complex model composed of two or more cosine curves that are harmonics of the so-called fundamental period, the method of multiple components provides three additional summary parameters: overall amplitude (one-half the difference between the maximum and minimum values of the best fitted curve, i.e. one-half the double global amplitude), and orthophase and bathyphase, i.e., peak and trough times, respectively, usually expressed as a lag relative to the time of awakening from nighttime sleep when the fundamental period is assumingly 24h. Assuming data are available from k different individuals or from a single individual evaluated several times during his/her follow-up, one can analyze each time series with the same multiple-component model; the estimated parameters can then be compared between individuals or between consecutive time-series from a given evaluated individual using included software for parameter testing.

3. Methods for time-series parameter estimation. Specific indices that are part of the system and reported for each evaluated individual include, but are not limited to, the following: descriptive statistics (mean, standard deviation, standard error, total range, 90% range, 50% range, interquartile range, minimum, maximum, median) for the complete time-series as well as for time-spans of clinical relevance (e.g., awake span, sleep span); sleep-time relative decline (percent decrease during sleep relative to awake mean); areas of excess (above an upper tolerance limit) and deficit (below a lower tolerance limit) and percent-times of excess and deficit for the entire investigated time, awake span, and sleep span; load (percentage of values above a given threshold); average real variability, morning surge, sleep-time fall, and ambulatory arterial stiffness index.

The system also comprises a risk-evaluation module **116** for assessing which clinical prognostic variables included in the database **110** or prognostic parameters **120** calculated by the analysis module **114**, as a proper combination, may better predict risk of occurrence of events or progression to a given disease. Based on actual outcomes derived from the database **110**, the risk-evaluation module **116** provides a model for multiple risk assessment based on the statistically significant prognostic variables/parameters. The risk-evaluation module 116 may be incorporated into the server **108**, implemented for instance in the server processor.

In many clinical applications the main objective is to evaluate, for any given individual, the individualized hazard ratio **122** of having an event (e.g., myocardial infarction, stroke, etc.) or developing a medical condition (e.g., sleep apnea, hypertension, diabetes, chronic kidney disease, etc.) within a given period of time. In such cases, for each specific endpoint of interest, e.g., total cardiovascular events, new-onset diabetes, sleep apnea, etc., the Cox proportional-hazard model, with adjustment for significant confounding variables, can be readily used to estimate hazard ratios **122** with 95% confidence intervals for events associated with each tested potential prognostic parameter, for example, either specific time-specified measurements of physiological variables of clinical interest for their prognostic value and/or characteristics of their sleep-wake predictable pattern. The model gives an expression for the hazard function h(t), i.e. the hazard at time $t$, for an individual with a given specification of a set of $p$ predictor variables, denoted by the vector $\mathbf{X}= (X_1,\dots,X_p)$:

$$ h(t,\mathbf{X}) = h_0(t)\exp\left(\sum_{i=1}^{p}\beta_i X_i\right) $$

Or, equivalently:

$$ \ln\left(\frac{h(t,\mathbf{X})}{h_0(t)}\right) = \sum_{i=1}^{p}\beta_i X_i $$

Where $h_0$ is the baseline hazard function, which depends on $t$, but does not involve X. On the other hand, the exponential expression involves X, but does not involve $t$. In other words, this assumes the predictors to be static, i.e., not dependent on time; the survival model, however, can be extended in order to reflect time-dependency. Even though the baseline hazard function $h_0(t)$ is unspecified, it is possible to estimate coefficients ($\beta$) and, using them, to evaluate the specific HR **122** either for a given individual or a stated population. The so-defined risk-evaluation model can also include interactions between predictor variables once their statistical significance can be documented.

The system further comprises an individualized multiple-disease quantitative risk assessment module **124**, which may be implemented for instance in the server processor or in a separate entity. Based on the risk-evaluation model performed in the risk evaluation module **116** and specified thresholds for each of the predictor variables incorporated in the model, a given individual's risk can be quantified numerically for each of the events or medical conditions under evaluation, including cardiovascular events, cerebrovascular events, and progression towards diabetes, chronic kidney disease, and sleep disorders. Additionally, the individualized risk assessment procedure allows quantification of the effect on the risk score of changes in any of the predictor variables. In particular, the use of this model applied to data from the same individual evaluated twice, i.e., before and after a specific therapeutic intervention, allows assessment of the effect of such intervention on the individualized risk score(s).

From the survival model implemented in the risk-evaluation module **116**, the hazard ratio **122** can be computed as the ratio between the models evaluated for two individuals (or for two groups of subjects to be compared if the variable is discrete). By computing such ratio, the baseline function disappears. For instance, if $\mathbf{X}^1$ and $\mathbf{X}^2$ are the data obtained for the significant predictor variables for subjects 1 and 2, respectively, one can estimate the hazard ratio as:

$$\hat{HR} = \frac{h(t, \mathbf{X}^1)}{h(t, \mathbf{X}^2)} = \exp\left(\sum_{i=1}^{p} \hat{\beta}_i (X_i^1 - X_i^2)\right)$$

If an ideal reference individual is defined as the one who has normative values (reference values) for all the predictor variables $X_1, ... X_p$, we can then compare any given test subject with this reference individual using this mathematical expression; this, in turn, allows estimation of the hazard ratio **122** for risk quantification of the test subject with respect to the defined reference values. The procedure does not require evaluating simultaneously the effect on risk of all predictor variables. Thus, one can easily quantify the potential effect on risk (either increase or decrease it) of changes in any single predictor variable included in the individualized risk-assessment model, simply by keeping constant the values of other additional confounding variables.

As an example of application, the modules for data analysis and graphical display of results applied for cardiovascular risk estimation are herein described. Previous findings indicate that, in particular, cardiovascular risk can be estimated on an individualized basis using a model that includes, as predictor variables, the sleeptime systolic BP mean (aSBP), the sleep-time relative systolic BP decline (dSBP), and the patient's characteristics of age, sex, diabetes, chronic kidney disease, and anemia, with several interactions. Given a test individual, with dSBP denoted $X_1$ and aSBP denoted $X_2$, his/her individualized HR can be calculated by comparison with an ideal reference individual. Let us assume both the test and the reference individual have the same sex and age, as well as the same conditions regarding presence/absence of diabetes, chronic kidney disease, and anemia. Assuming also the values of the BP-related predictor variables dSBP and aSBP for the reference individual are the customary present thresholds for identification of the dipper/non-dipper BP pattern (dSBP=10%) and sleeptime hypertension (aSBP=12 cmHg), respectively, then:

$$\hat{HR} = \exp\left(\hat{\beta}_1 (X_1 - 10) + \hat{\beta}_2 (X_2 - 12)\right)$$

While some of the predictor variables of the model are not modifiable by treatment, i.e., sex or age, others might well change in time due to therapeutic intervention, in particular those derived from ambulatory BP monitoring (e.g., aSBP and dSBP). Accordingly, the described procedure allows numerical quantification of the changes in any given subject risk score associated with specified target treatment-induced changes in the modifiable predictors, e.g., a 1 cmHg decrease in aSBP, or a 5% increase in dSBP. Such a potential treatment-induced modification in estimated risk can be easily visualized graphically with a contour plot. Individualized risk scores for additional conditions, such as diabetes or chronic kidney disease, can be estimated for the very same test subject using the same approach with the properly tested model that includes relevant and statistically significant predictor variables for each condition of interest.

For any given predicting model, where risk can be described by only two therapeutically modifiable parameters (e.g., aSBP and dSBP), the individualized quantification of risk can be graphically represented as follows:

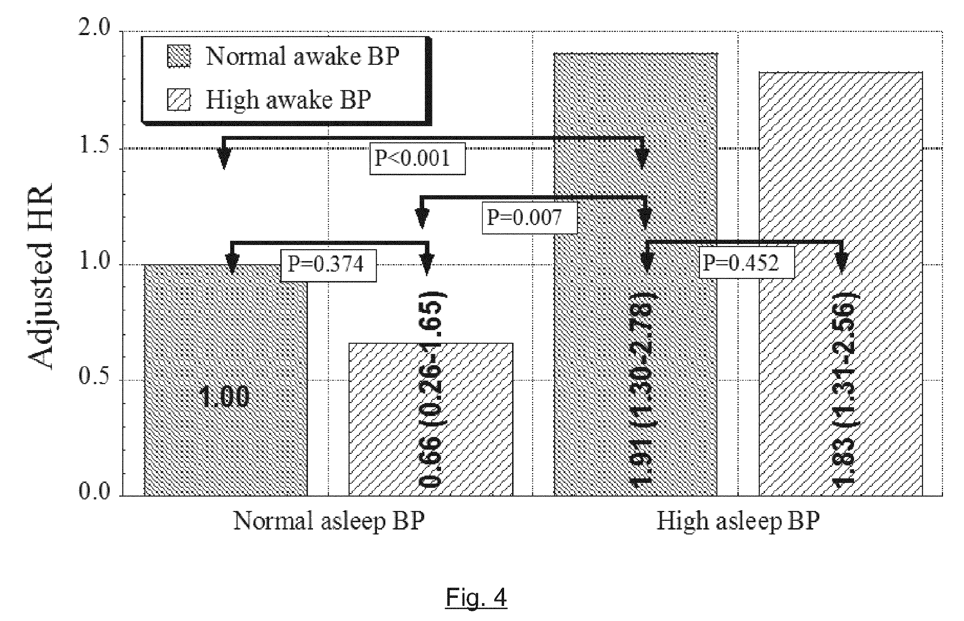

a) The risk function (hazard ratio **122**), as it depends on only two modifiable predictor variables, will be represented in a three-dimensional plot, as shown in Figure 4. The specific shape of risk surface **204** depends on the predictor variables **206** involved (systolic awake-asleep ratio and asleep systolic mean, in the case shown in Figure 4) and on the risk function obtained from the information collected in the database **110**. The individualized risk score **126** of any given test subject will be obtained by calculating the specific point on the risk surface **204** that corresponds with the values measured for each of the predictor variables **206** for that particular test subject.

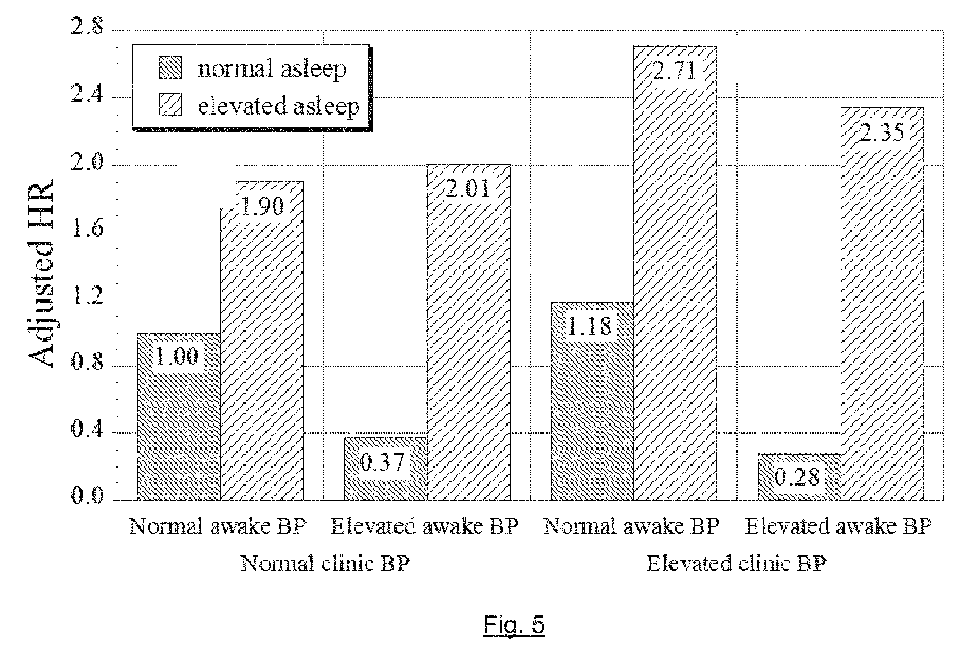

b) Since the translation to a two-dimensional graph does not allow a clear view of the specific values associated with a three-dimensional function, a simple alternative is to reduce the risk plot to a two-dimensional graphic and to represent risk score ranges with different colors. Such graphical representation is shown in Figure 5, where the risk score **126** for the test subject is easily identifiable simply by knowing the measured values of the predictor variables **206**. The value of the individualized hazard ratio **122** can be determined by the color associated with the location of the test subject according to the provided scale. Although the risk function is a continuous variable, the scale has been converted into a discrete variable to simplify visual inspection and interpretation of the results. Using such a graphical representation of the risk function, the potential effects on the individualized risk score of changes (due to treatment, aging, or any other factors) in the modifiable predictor variables **206** can be readily visualized, this allowing the design of personalized therapeutic strategies for proper risk reduction.

Experimental Tests and Results

A validation study was conducted to evaluate the performance of a particular embodiment of the system and computer-implemented method for individualized risk assessment, specifically as example for development of new-onset diabetes. Sleep-time hypertension and the non-dipper and riser BP patterns are highly prevalent in patients with type 2 diabetes. These conditions, reflecting altered BP regulation, can only be determined by ABPM and they have been consistently associated with the increased CVD risk of patients with than without diabetes. However, whether alterations in BP determined by ABPM indeed provide prognostic value for predicting the development of diabetes has never before been prospectively investigated. Most important, whether or not the risk of developing diabetes can be modified by specifically targeting potential prognostic risk markers, e.g., the potential reduction in the risk of diabetes associated with decreasing an elevated sleep-time BP mean, is unknown. We evaluated 2656 subjects without diabetes, 1292 men/1364 women, 50.6±14.3 years of age, with baseline ambulatory BP ranging from normotension to sustained hypertension according to established diagnostic criteria based on ABPMan awake BP mean of ≥135/85 mmHg for SBP/DBP, and/or an asleep BP mean ≥120/70 mmHg. The subjects were participants of the MAPEC study without diabetes at the time of recruitment. At baseline and yearly (more frequently if hypertension treatment required adjustment based on ABPM criteria) thereafter, ambulatory BP and physical activity (wrist actigraphy to accurately derive the awake and asleep BP means on an individual basis) were simultaneously monitored for 48h, instead of the most common 24h monitoring span, in order to increase accuracy and reproducibility of the findings. The Cox proportional-hazard model, adjusted for significant confounding variables, was used to estimate HR for the risk of developing diabetes associated with clinic BP measurement and ABPM-derived parameters, including the 48h, awake, and asleep BP means, the sleep-time-relative BP decline, and dipping classification.

During a 5.6-year median follow-up, 190 participants developed diabetes. The asleep, but not awake, SBP mean was a highly significant predictor of new-onset diabetes in a Cox proportional-hazard model adjusted for the significant confounding variables of age, waist perimeter, glucose, and chronic kidney disease (for each 1-SD elevation, hazard ratio 1.30, [95%CI: 1.13-1.48] for asleep SBP, P<0.001; 1.12 [0.97-1.29] for awake SBP, P=0.128). Most important, when the asleep SBP mean is adjusted for the awake SBP mean, only the former significantly predicts new-onset diabetes. To further investigate the clinical relevance of the awake and asleep BP means on the risk of diabetes, the participants of the MAPEC study were divided into four groups according to BP level at the final evaluation, i.e., normal or elevated, using established ABPM thresholds of 135/85 mmHg for the awake SBP/DBP means and of 120/70 mmHg for the asleep SBP/DBP means, independent of clinic BP. Figure 4 shows the results of this analysis that indicate: (i) equivalent adjusted HR for diabetes of participants with normal asleep BP whether the awake BP mean is normal or elevated (P=0.374); (ii) equivalent HR in hypertensive patients with elevated asleep BP, independent of awake BP mean (P=0.452); and (iii) higher adjusted HR of diabetes in patients with elevated asleep BP mean than participants with normal asleep BP, whether the awake BP mean is below or above 135/85 mmHg (P<0.001). Exploration of the combined contribution to the risk of diabetes of multiple BP parameters indicated clinic SBP had no predicting value when corrected by asleep SBP mean (HR=1.10 [0.94-1.29], P=0.210). Indeed, when each of the four groups of participants categorized by awake and asleep BP means in Figure 4 were further divided according to either normal or elevated clinic BP measurements using the currently accepted 140/90 mmHg thresholds, as shown in Figure 5 the risk of diabetes was significantly higher in the four groups of patients with elevated asleep BP mean, regardless of either clinic BP or the ABPM-derived awake BP mean being normal or elevated, than in the other four groups of patients with normal sleep-time BP mean.

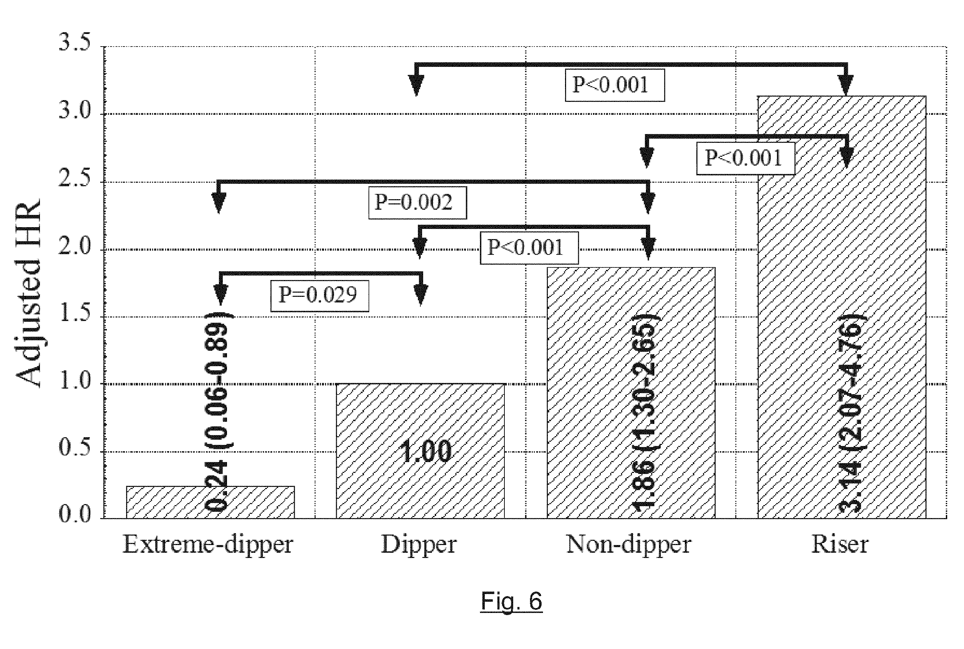

Additionally, analyses of changes in BP during follow-up (difference for any tested BP parameter between the values obtained upon recruitment and at the final evaluation) revealed a 30% reduction in the risk of developing diabetes for each standard deviation (SD) decrease in asleep SBP mean (P<0.001), independent of changes in daytime clinic or ABPM-derived awake BP mean. Only the increase in sleep-time relative SBP mean towards more normal dipper BP patterning improved the prognostic value of sleep-time SBP (HR=0.82 [95%CI: 0.70-0.96], P=0.013). Indeed, as a single analyzed variable, the diminished sleep-time relative BP decline was a highly significant predictor of new-onset diabetes in a Cox proportional-hazard model adjusted for the significant influential variables of age, waist perimeter, glucose, and chronic kidney disease (for each 1-SD elevation, HR=0.77, [95%CI: 0.68-0.88], P<0.001). Based on the baseline ABPM evaluation per participant, the adjusted HR was similar in extreme-dippers (sleep-time relative BP decline ≥20%) and dippers (sleep-time relative BP decline ≥10% and <20%; P=0.882), but significantly greater in non-dippers (sleep-time relative BP decline <10% and ≥0%; P=0.002) and risers (sleep-time relative BP decline <0%; P<0.001). Based on the data of the last 48h ABPM profile per participant, as shown in Figure 6, compared to dippers the adjusted HR of new-onset diabetes was lower in extreme-dippers (P=0.029) and significantly higher in non-dippers and risers (P<0.001). According to this prospective evaluation, sleep-time SBP mean, but not daytime clinic BP measurement or ABPM-derived awake BP mean, is a highly significant and independent prognostic marker of the development of new-onset diabetes. Alteration in sleep-time BP regulation, highly frequent in diabetes, seems to precede diabetes, rather than to be a consequence of this condition. These findings indicate ABPM is a clinical necessity to accurately detect abnormal sleep-time BP and evaluate the risk of progression to diabetes. More important, decreasing asleep SBP and increasing sleep-time relative SBP decline are significant independent predictors of reduced risk of developing diabetes.

Certain specific details in the above description and figures provide a thorough understanding of various embodiments disclosed. Certain well-known details often associated with computing, computer-implemented methods and associated systems, and software technology are not set forth in the following disclosure to avoid unnecessary obscuring the various disclosed embodiments. Further, those of ordinary skill in the relevant art will understand that they can practice other embodiments without one or more of the details described below. Aspects of the disclosed embodiments may be implemented in the general context of computer-executable instructions, such as program modules, being executed by a computer, computer server, or device containing a processor. Generally, program modules include routines, programs, objects, components, data structures, etc., that perform particular tasks or implement particular abstract data types. Aspects of the disclosed embodiments may also be practiced in distributed computing environments where tasks are performed by remote processing devices that are linked through a communications network. In a distributed computing environment, program modules may be located in both local and remote storage media including memory storage devices. Those skilled in the art will appreciate that given the description of the modules comprising the disclosed embodiments provided in this specification, it is a routine matter to provide working systems that work on a variety of known and commonly available technologies capable of incorporating the features described herein.

While particular embodiments have been described, it is understood that, after learning the techniques contained in this disclosure, modifications and generalizations will be apparent to those skilled in the art without departing from the spirit of the disclosed embodiments. It is noted that the foregoing embodiments and examples have been provided merely for the purpose of explanation and are in no way to be construed as limiting. While the method and/or system has been described with reference to various embodiments, it is understood that the words that have been used herein are words of description and illustration, rather than words of limitation. Further, although the system has been described herein with reference to particular means, materials, and embodiments, the actual embodiments are not intended to be limited to the particulars disclosed herein; rather, the system extends to all functionally equivalent structures, methods and uses, such as are within the scope of the appended claims. Those skilled in the art, having the benefit of the teachings of this specification, may effect numerous modifications thereto and changes may be made without departing from the scope and spirit of the disclosed embodiments in its aspects.

**Claims**

1. A system for individualized multiple-disease quantitative risk assessment, the system (112) comprising:

   - a database (110) storing clinical data of at least one patient, said clinical data comprising prognostic variables including at least a plurality of biological signals (118) measured on the at least one patient;
   - an analysis module (114) for processing the biological signals (118) stored in the database (110) for a given patient through at least one statistical method and analysis technique, to automatically calculate prognostic parameters (120) of diagnostic relevance for said patient;
   - a risk-evaluation module (116) for estimating, using a risk-evaluation model that considers predictor variables among the prognostic variables stored in the database (110) and the prognostic parameters (120) calculated by the analysis module (114), an individualized hazard ratio (122) of having an event or developing a medical condition within a given period of time for the given patient; and
   - an individualized multiple-disease quantitative risk assessment module (124) for computing, based on the risk-evaluation model performed in the risk evaluation module (116) and specified thresholds for each of the predictor variables incorporated in the risk-evaluation model, a quantification of the risk (126) for the given patient for each of the events or medical conditions under evaluation.

2. System according to claim 1, wherein the statistical method and analysis technique performed in the analysis module (114) includes methods for computation of time-specified tolerance intervals and bands.

3. System according to claim 2, wherein for a given lower and upper limit of a time-varying tolerance interval of the biological signal under analysis, diagnosis of disease for a given patient is established in the analysis module (114) by calculating the total area of the patient's biological signal measurements above or below the lower and upper limits during the entire 24 hour period.

4. System according to claim 1, wherein the statistical method and analysis technique performed in the analysis module (114) includes methods for time-series periodic evaluation that provide sensitivity diagnostic endpoints.

5. System according to claim 1, wherein the statistical method and analysis technique performed in the analysis module (114) includes methods for time-series parameter estimation.

6. System according to any of previous claims, wherein the risk-evaluation module (116) uses a Cox proportional-hazard model to estimate the hazard ratio (122) for a given patient.

7. System according to claim 6, wherein the individualized hazard ratio (122) is computed as the ratio between the risk-evaluation models evaluated for the given patient and a reference individual with normative values for all the predictor variables.

8. System according to any of previous claims, wherein the clinical data stored in the database (110) includes laboratory data derived from blood, urine or saliva.

9. System according to any of previous claims, wherein the clinical data stored in the database (110) includes current treatment of each patient.

10. System according to any of previous claims, wherein the clinical data stored in the database (110) includes personal medical history, anthropometric information and personal habits.

11. System according to any of previous claims, wherein the events or medical conditions under evaluation include at least any of the following: cardiovascular events, cerebrovascular events, progression towards diabetes, renal disease and/or sleep disorders.

12. System according to any of previous claims, the system (112) further comprising a server (108) running a web-enabled graphical user interface to enable a user (100) to authenticate and upload clinical data of different patients.

13. Computer-implemented method for individualized multiple-disease quantitative risk assessment, comprising:

- retrieving, from a database (110) storing clinical data of at least one patient comprising prognostic variables which include at least a plurality of biological signals (118) measured on the at least one patient, biological signals (118) for a given patient;
- processing the retrieved biological signals (118) through at least one statistical method and analysis technique, to automatically calculate prognostic parameters (120) of diagnostic relevance for said patient;
- estimating, using a risk-evaluation model that considers predictor variables among the prognostic variables stored in the database (110) and the prognostic parameters (120) calculated by the analysis module (114), an individualized hazard ratio (122) of having an event or developing a medical condition within a given period of time for the given patient; and
- computing, based on the risk-evaluation model performed in the risk evaluation module (116) and specified thresholds for each of the predictor variables incorporated in the risk-evaluation model, a quantification of the risk (126) for the given patient for each of the events or medical conditions under evaluation.

14. Computer-implemented method according to claim 13, comprising dynamically reevaluating the risk assessment for a given patient as new clinical data for said patient is stored in the database (110).

15. Computer program product for individualized multiple-disease quantitative risk assessment, wherein the computer program product comprises at least one computer-readable storage medium comprising a set of instructions stored therein which, when executed by a processor, causes the processor to:

- retrieve, from a database (110) storing clinical data of at least one patient comprising prognostic variables which include at least a plurality of biological signals (118) measured on the at least one patient, biological signals (118) for a given patient;
- process the retrieved biological signals (118) through at least one statistical method and analysis technique, to automatically calculate prognostic parameters (120) of diagnostic relevance for said patient;
- estimate, using a risk-evaluation model that considers predictor variables among the prognostic variables stored in the database (110) and the prognostic parameters (120) calculated by the analysis module (114), an individualized hazard ratio (122) of having an event or developing a medical condition within a given period of time for the given patient; and
- compute, based on the risk-evaluation model performed in the risk evaluation module (116) and specified thresholds for each of the predictor variables incorporated in the risk-evaluation model, a quantification of the risk (126) for the given patient for each of the events or medical conditions under evaluation.

Fig. 1

Fig. 2

EP 3 096 253 A1

Fig. 3

Fig. 4

Fig. 5

Fig. 6

EP 3 096 253 A1

Europäisches
Patentamt

European
Patent Office

Office européen
des brevets

# EUROPEAN SEARCH REPORT

Application Number

EP 15 16 8068

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/050186 A2 (CATERPILLAR INC [US]; GRICHNIK ANTHONY J [US]; SESKIN MICHAEL [US]) 3 May 2007 (2007-05-03) <br> * page 1 - page 3, line 7 * <br> * page 5, line 16 - line 21 * <br> * page 6, line 7 - page 7, line 20 * <br> * page 11, line 8 - page 12, line 24 * <br> * page 14, line 7 - page 15, line 7 * <br> ----- | 1-15 | INV. <br> G06F19/00 |
| X | US 2013/262357 A1 (AMARASINGHAM RUBENDRAN [US] ET AL) 3 October 2013 (2013-10-03) <br> * paragraph [0034] - paragraph [0041]; figure 3 * <br> ----- | 1-15 | |
| X | US 2005/203773 A1 (SOTO GABRIEL E [US] ET AL) 15 September 2005 (2005-09-15) <br> * paragraph [0089] * <br> * paragraph [0093] - paragraph [0094] * <br> * paragraph [0108] - paragraph [0110] * <br> * paragraph [0155] - paragraph [0156] * <br> ----- | 1-15 | |
| A | Anonymous: "Proportional hazards model", Wikipedia, 25 December 2014 (2014-12-25), XP055220717, Retrieved from the Internet: URL:https://en.wikipedia.org/w/index.php?title=Proportional_hazards_model&oldid=639622856 [retrieved on 2015-10-14] <br> * the whole document * <br> ----- | 6 | TECHNICAL FIELDS SEARCHED (IPC) <br><br> G06F |
| A | WO 2009/091583 A1 (MASSACHUSETTS INST TECHNOLOGY [US]; GUTTAG JOHN V [US]; SYED ZEESHAN H) 23 July 2009 (2009-07-23) <br> * page 19, lines 8-17 * <br> ----- | 6 | |

The present search report has been drawn up for all claims

| Place of search | Date of completion of the search | Examiner |
|---|---|---|
| Munich | 19 October 2015 | Eichenauer, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 16 8068

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2015

| Patent document cited in search report | | Publication date | Patent family member(s) | | Publication date |
|---|---|---|---|---|---|
| WO 2007050186 | A2 | 03-05-2007 | CN 101297297 A | | 29-10-2008 |
| | | | EP 1941410 A2 | | 09-07-2008 |
| | | | US 2007094048 A1 | | 26-04-2007 |
| | | | US 2007179769 A1 | | 02-08-2007 |
| | | | WO 2007050186 A2 | | 03-05-2007 |
| US 2013262357 | A1 | 03-10-2013 | NONE | | |
| US 2005203773 | A1 | 15-09-2005 | US 2005203773 A1 | | 15-09-2005 |
| | | | US 2011093288 A1 | | 21-04-2011 |
| | | | US 2013132323 A1 | | 23-05-2013 |
| | | | US 2014046682 A1 | | 13-02-2014 |
| | | | WO 2006096603 A2 | | 14-09-2006 |
| WO 2009091583 | A1 | 23-07-2009 | US 2009192394 A1 | | 30-07-2009 |
| | | | US 2013046193 A1 | | 21-02-2013 |
| | | | US 2014296724 A1 | | 02-10-2014 |
| | | | WO 2009091583 A1 | | 23-07-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8428965 B2